Europäisches Patentamt

European Patent Office

Office européen des brevets

(19)

(11) Publication number: **0 015 585**

**B1**

(12) **EUROPEAN PATENT SPECIFICATION**

(45) Date of publication of patent specification: **09.01.85**

(51) Int. Cl.⁴: **B 01 J 31/06, B 01 J 33/00**

(21) Application number: **80101243.6**

(22) Date of filing: **11.03.80**

(54) Liquid-gas catalytic reaction process.

(30) Priority: 12.03.79 JP 29754/79
12.03.79 JP 29755/79
12.03.79 JP 29756/79
12.03.79 JP 29757/79
12.03.79 JP 29758/79
12.03.79 JP 29759/79

(43) Date of publication of application:
**17.09.80 Bulletin 80/19**

(45) Publication of the grant of the patent:
**09.01.85 Bulletin 85/02**

(84) Designated Contracting States:
**BE CH DE FR GB IT NL**

(56) References cited:
CA-A- 959 821
FR-A-1 311 864
FR-A-2 255 103
US-A-2 722 504
US-A-4 025 560

(73) Proprietor: **Hitachi, Ltd.**
5-1, Marunouchi 1-chome
Chiyoda-ku Tokyo 100 (JP)

(72) Inventor: **Mori, Toshikatsu**
1047, Moriyama
Hitachi Ibaraki (JP)
Inventor: **Kumagai, Teruo**
161, Ishinazaka
Hitachi Ibaraki (JP)
Inventor: **Takeuchi, Seiji**
811-3, Isobe
Hitachiouta Ibaraki (JP)
Inventor: **Takeuchi, Masato**
2428-6, Kouya
Katsuta Ibaraki (JP)
Inventor: **Kato, Akira**
161, Ishinazaka Hitachi
Ibaraki (JP)
Inventor: **Matsuda, Shinpei**
1722-7, Shirakata Tohkai-mura
Naka Ibaraki (JP)
Inventor: **Yamashita, Hisao**
2374-48, Ohkubo, Hitachi
Ibaraki (JP)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**O 015 585**

(58) References cited:

**The file contains technical information submitted after the application was filed and not included in this specification**

(74) Representative: **Strehl, Peter, Dipl.-Ing. et al Strehl, Schübel-Hopf, Schulz Patentanwälte Widenmayerstrasse 17 Postfach 22 03 45 D-8000 München 22 (DE)**

# 0 015 585

## Description

The present invention relates to a catalytic process for reducing organic and inorganic ions and molecules which comprises contacting said ions or molecules present in an aqueous liquid reaction system in the presence of a solid hydrophobic catalyst with a reducing gaseous substance hardly or not soluble in said aqueous liquid reaction system.

As methods for reducing ions or molecules in an aqueous solution, there are known (1) a process in which a water-soluble reducing agent such as sodium sulfite, sodium sulfide or acidic stannous chloride is used, (2) a process in which a powder of a metal producing hydrogen in the nascent state under acidic or alkaline conditions, such as zinc, aluminum or tin, is used, (3) a process in which a water-soluble reducing gas such as hydrogen sulfide or sulfurous acid gas is used and (4) a process in which a reducing gas hardly soluble in water, such as hydrogen or carbon monozide is used. Although these techniques are practically used they involve several defects. For example, in the processes (1), (2) and (3), undesirable by-products are formed in the liquid reaction mixtures, reaction vessels are readily corroded, and the reducing agents used are expensive, Furthermore, in the process (4), since the reducing gas used is hardly soluble in water, high temperatures and high pressures are necessary for completion of the reaction. As means for eliminating this defect and increasing the reaction rate, there has been proposed a process in which a homogeneous catalyst system is used. According to this process, however, no substantial improvement is attained and the separation of the catalyst from the reactants is very difficult. Furthermore, even if heterogeneous catalysts are used, since conventional solid catalysts are hydrophilic, the catalyst surface is covered with water and the reducing gas is hardly adsorbed on the catalyst surface, with the result that no substantial catalytic effect can be attained.

Various processes for promoting certain reactions by using water-repellent catalysts are already known.

There has been proposed a process for concentrating heavy water by the isotope exchange reaction between water and hydrogen in the presence of a platinum type catalyst which has been rendered water-repellent. For rendering such catalyst water-repellent, a hydrophilic $Pt-Al_2O_3$ or Pt-active carbon catalyst is coated with a silicone oil (JP—A—32800/76) or with a polytetrafluoroethylene resin (JP—A—41195/76 and JP—A—155492/75).

JP—A—41195/76 describes a process in which hydrogen isotopes are concentrated by using a catalyst comprising an element of the group VIII of the Periodic Table supported on the surface of a polytetrafluoroethylene carrier.

JP—A—156297/77 discloses a double-temperature exchange process in which hydrogen isotopes are exchanged between water and $H_2$ by contacting them in a countercurrent manner with a catalyst having a film of a hydrophobic polymer.

JP—A—4197/78 discloses a process in which a reaction is carried out in a mixed bed comprising a hydrophobic catalyst including a supported noble metal and a hydrophilic carrier.

US—A—4,061,724 discloses a process in which organic materials are selectively adsorbed from water by using silica which has been rendered hydrophobic.

CA—A—958,821 and CA—A—959,628 relate to oxidation and reduction processes using a catalyst whose carrier or substrate is conductive for electrons. This known process may be used for different oxidation reactions, such as the oxidation of phenols by air, and reduction reactions, such as the reduction of potassium permanganate by hydrogen, in which the reactants form a gas-liquid or liquid-liquid system which is in contact with the solid catalyst. For the known catalyst is is essential that the catalyst carrier itself is an electronically conductive material which is not hydrophobic per se, but which is rendered hydrophobic by a treatment with a hydrophobic material. The teaching of CA—A—958,821 therefore suggests that for the desired reaction it is necessary that the carrier is effective as an electronically conductive substrate, although it may be partially made hydrophobic at the surface.

From US—A—4,025,560 a process for the exchange of hydrogen isotopes between streams of gaseous hydrogen and liquid water in the prexence of a catalyst consisting of a hydrophobic carrier and a catalytically active metal, e.g. platinum, is known. Although the carrier used in this known process may be hydrophobic without any surface treatment, there is no indication about a specific critical surface tension. Furthermore, the known catalyst is used in a process for the exchange of hydrogen isotopes, which is not comparable with a catalytic process for reducing organic and inorganic ions and molecules.

The problem on which the present invention is based is to provide a process by which ions and molecules dissolved in an aqueous reaction system may be reduced by a gaseous reducing substance, which is essentially insoluble in the aqueous liquid reaction system, with high effectiveness and without the necessity to apply a high pressure of the gaseous medium.

According to the invention there is provided a catalytic process for reducing organic and inorganic ions and molecules which comprises contacting said ions or molecules present in an aqueous liquid reaction system in the presence of a solid hydrophobic catalyst with a reducing gaseous substance hardly or not soluble in said aqueous liquid reaction system. The process is characterized in that said catalyst comprises as constituents a catalytic component and, as a carrier for the catalytic component, a porous, water-repellent organic polymeric substance having fine pores and a large specific surface

3

area and in that said catalyst has a critical surface tension of lower than $2 \cdot 10^{-2}$ N·m$^{-1}$ (20 dyn/cm) as measured at 20°C and a contact angle to water larger than 60°.

According to the present invention a heterogeneous system capable of advancing the achieved reaction very easily is formed when reacting a substance in a liquid reaction system with a gas hardly or not soluble in the liquid reaction system in the presence of a catalyst as defined above, which is non-compatible with the liquid.

In the instant specification, by the term "non-compatible with the liquid", it is meant that the surface of the solid has such a property that the surface of the solid is not completely wetted with the liquid and only a discontinuous film of the liquid is formed on the surface of the solid.

According to the present invention, by virtue of this specific property of the solid catalyst, three phases, that is, a liquid phase, a gas phase and a solid phase, are simultaneously formed on the surface of the catalyst, and it is considered that the reaction gas is allowed to readily arrive at active sites of the catalyst and advance of the reaction is promoted. The present invention will now be described in detail.

The water-repellent solid catalyst that is used in the process of the present invention comprises a water-repellent carrier, and as used porous particles and shaped articles of various polymers such as polytetrafluoroethylene, polyethylene, polypropylene, polystyrene, cross-linked polystyrene and polymethyl methacrylate.

By the process of the present invention organic and inorganic ions and molecules can be reduced. As examples of the organic substance, there can be mentioned hydrocarbons such as olefins and aromatic compounds, oxygen-containing compounds such as aldehydes and organic acids, e.g., acetic acid, nitrogen-containing compounds such as nitro compounds, sulfur-containing compounds such as mercaptans and sulfides, e.g., thiophene, and organic compounds containing halogens, metals, phosphorus or the like. As the inorganic substance, there can be mentioned, for example, ions of metals having two or more different valencies, such as $Fe^{3+}$, $Cu^{2+}$, $Cr^{5+}$ and other transition metal ions, ions of metal oxyacids such as $VO^{2+}$, $CrO_4^{2-}$ and $WO_4^{2-}$, nitrogen-containing ions such as $NO_3^-$ and $NO_2^-$, sulfur-containing ions such as $SO_4^{2-}$ and $S_2O_6^{2-}$, and complex compounds of metals.

As typical examples of the reducing gas that is used in the present invention, there can be mentioned hydrogen, carbon monoxide, nitrogen monoxide, methane and nitrogen suboxide. These gases may be used singly or in combination with diluent gases. A mixture containing a plurality of reducing gases, such as synthesis gas, may be used in the present invention without any trouble.

At least a part of the surface of the water-repellent catalyst that is used in the present invention has such a water-repellent property that in an aqueous solution, all the surface of the catalyst is not completely wetted or covered with the aqueous solution and the reducing gas can be contacted with or adsorbed on the surface of the catalyst. Such water-repellent catalyst may be prepared according to the above-mentioned methods.

According to a preferred embodiment of the present invention at least a part of the surface of the catalyst is water-repellent and oil-repellent, resulting in the fact, that the surface of the catalyst is not completely covered with water, solvent or reaction product but a gas phase can be formed on the surface of the catalyst. As the carrier of the water-repellent and oil-repellent catalyst, there can be used, for example, particles and shaped articles of organic polymers such as polytetrafluoroethylene, polyethylene, cross-linked polystyrene and polymethacrylate, and particles and shaped articles coated with these materials.

As will be apparent from its name, the water-repellent and oil-repellent catalyst has no affinity to either water or an oil.

When a conventional hydrophilic or water-repellent catalyst is used for the reduction of an organic substance dissolved in water with a gaseous organic reducing substance, the surface of the catalyst is partially wetted with water or the dissolved organic substance, and the gaseous reactant cannot arrive at the surface of the catalyst. The speed of advance of the reaction therefore is very low.

On the other hand, when the water-repellent and oil-repellent catalyst of the present invention is applied to such reactions, since the catalyst has a property of strongly repelling water and organic substances the gaseous reactant which is hardly soluble in water can easily arrive at the surface of the catalyst and the reactions are remarkably promoted. Accordingly, the water-repellent and oil-repellent catalyst of the present invention is very effective for reactions of organic substances having a relatively large surface tension. Therefore, in order to utilize the activity of the water-repellent and oil-repellent catalyst most efficiently, it is necessary to select a catalyst having a critical surface tension lower than the surface tension of the starting reaction liquid and the surface tension of the reaction product liquid. The critical surface tension of the catalyst is lower than $2 \cdot 10^{-2}$ N·m$^{-1}$ (20 dyn/cm) as measured at 20°C. Furthermore, the contact angle of the catalyst to water is larger than 60° and the catalyst carrier should have fine pores and a large specific surface area. A catalyst formed by supporting a catalytic active component on a carrier can be additionally treated with a water-repellent agent in order to enhance the hydrophobic properties. Thus, a water-repellent and oil-repellent catalyst is formed.

Catalytic components customarily used for gas phase reaction can be used for the catalytic active component of the water-repellent and oil-repellent catalyst of the present invention. As typical examples of the catalytic component, there can be mentioned elements of the group VIII of the Periodic Table (Fe, Co, Ni and metals of the platinum group such as Pt, Pd, Ru, Rh, Ir and Os), elements of the group V of the Periodic Table (such as V, Nb, As and Bi), elements of the group VIA (such as Cr, Mo and W), elements of the group IV (such as Ti, Sn and Pb), elements of the group IB (such as Cu, Ag and Au) and elements of the group IIB (such as Zn, Cd and Hg). These elements are used for the reaction in the form of metals, oxides, sulfates and chlorides. These active components are supported singly or in combination by mixing, lamination or impregnation. The active metal is supported in an amount of 0.01 to 10% by weight as the metal atom. The shape of the water-repellent and oil-repellent catalyst is not particularly critical in the present invention. For example, fine particles having a size of 0.01 to 0.1 mm, spherical, columnar and cylindrical pellets having a size of 1 to 10 mm and honey-comb and plate-like shaped articles having a large size can be used.

Ordinary reaction vessels of the fixed bed type, moving bed type, fluidized bed type and suspended bed type can be used for reducing substances in aqueous solutions according to the process of the present invention. Furthermore, contact of an aqueous solution with a reducing gas may be performed in a countercurrent manner or a concurrent manner or may be performed batchwise. The shape of the water-repellent catalyst is not particularly ciritical in the present invention. For example, fine particles having a size of 0.01 to 1 mm, spherical, columnar and cylindrical pellets having a size of 1 to 10 mm and honey-comb and plate-like shaped articles having a larger size can be used.

When the process of the present invention is actually carried out, since an aqueous solution is repelled by the water-repellent catalyst, the dispersion state of the aqueous solution often becomes bad, and a particular contrivance is required in such case, especially when a fixed bed is used. In such case, good results are obtained when a catalyst bed is formed by mixing a water-repellent catalyst with a hydrophilic catalyst or hydrophilic carrier.

In practising the reduction process of the present invention, the lower limit of the reaction temperature is the temperature of solidification of water, and the upper limit of the reaction temperature is determined depending on the heat resistance of the water-repellent substance. The reaction can be carried out under a pressure of 0.1 to 1000 bars, preferably 0.5 to 100 bars.

Examples for the preparation of the water-repellent and oil-repellent catalyst of the present invention will now be described.

Catalyst Preparation Process (I)

Porous polytetrefluoroethylene having a square shape having a side of 5 mm and a thickness of 1 mm was dried at 120°C for 2 hours and then deaerated. Then, the polytetrafluoroethylene was impregnated with a liquid mixture of chloroplatinic acid and acetone, dried and reduced for 3 hours in a hydrogen stream at 200 to 240°C to form a catalyst having Pt supported in an amount of 0.5% by weight based on the carrier.

Catalyst Preparation Process (II)

The catalyst prepared in the process (I) was dipped in a polytetrafluoroethylene suspension (marketed under tradename "Polyflon Liquid") to render the catalyst water-repellent.

Catalyst Preparation Process (III)

The catalyst prepared in the process (I) was immersed in a solution of copper sulfate having a concentration of 0.1 mole/l and wet-reduced for 1 hour while introducing hydrogen gas into the solution. The reduced catalyst was dried and then similarly reduced in a solution of chloroplatinic acid. In the resulting catalyst, Pt and Cu were supported in amounts of 0.6% by weight and 0.5% by weight, respectively, based on the carrier.

Catalyst Preparation Process (IV)

The catalyst prepared in the process (I) was rendered water-repellent by dipping it in Polyflon Liquid.

The above-mentioned processes (I) to (IV) are ordinary processes for preparing water-repellent and oil-repellent catalysts. A carrier is selected from organic polymeric materials such as mentioned above. An active component is selected from the above-mentioned active metals. As described above with respect to the preparation process (III), different metals may be supported by lamination or mixing. Furthermore, the hydrophilic property imparted by deposition of an active metal may be reduced by an additional treatment of the catalyst with a water-repelling agent described above with reference to the preparation processes (II) and (IV) or with silicone compounds.

The present invention will now be described in detail with reference to the following Examples and Comparative Examples.

Example 1

This Example illustrates reduction of a ferric-ETDA complex ion [$Fe^{(III)} \cdot Y^-$] with hydrogen.

A carrier shown in Table 1 was impregnated with a liquid formed by diluting Polyfon Liquid with water at a dilution ratio of 50, dried at 100°C for 20 hours, impregnated with an ethyl alcohol solution of chloroplatinic acid or palladium chloride, dried at 100°C for 5 hours and activated by reduction with hydrogen at 180°C. An aqueous solution containing 0.1 mole/l of $Fe^{(III)} \cdot Y^-$ ions, which was formed by dissolving commercially available iron ethylenediamine-tetra-acetate (NaFeY·2H$_2$O) in water, was used. An absorbing bottle equipped with a ball filter was charged with 100 ml of this solution and 10 ml of the above catalyst was suspended in the solution, and hydrogen was fed at a rate of 100 ml/min in the form of fine bubbles and reduction of Fe$^{(III)}$·Y$^-$ ions was conducted for 1 hour. At this reaction, it was confirmed that the catalyst floated and was predominantly present in the liquid surface protion, and that bubbles of hydrogen adhered to the catalyst surface. The reaction temperature was set by placing the absorbing bottle in a thermostat tank maintained at 80°C. The reaction pressure was controlled to atmospheric pressure. The obtained results are shown in Table 1.

Table 1

| Carrier | Active Component | Supported Amount (% by weight) | Ratio (%) of Reduction of Fe$^{(III)}$·Y$^-$ Ions |
|---|---|---|---|
| Cation Exchange Resin | Pd | 0.5 | 90 |

Comparative Example 1

Reduction of Fe$^{(III)}$·Y$^-$ ions was carried out in the same manner as described in Example 1 except that the carrier was not impregnated with Polyflon Liquid. In case of each carrier, the ratio of reduction of Fe$^{(III)}$·Y$^-$ ions was lower than 15%.

Example 2

This Example illustrates the reduction of sodium sulfate (Na$_2$SO$_4$) with hydrogen.

A glass reaction vessel having an inner capacity of 1 liter was charged with 100 ml of an aqueous solution of sodium sulfate having a concentration of 0.1 mole/l and 20 ml of a catalyst formed by supporting 1% by weight of platinum on a carrier obtained by slicing a polytetrafluoroethylene tube having an outer diameter of 5 mm and and inner diameter of 3 mm was added to the aqueous solution. Hydrogen was fed into the solution at a feed rate of 200 ml/min and reaction was carried out at a temperature of 150°C under a pressure of 5 bars (kg/cm$^2$) G for 2 hours. The ratio of reduction of sodium sulfate to sodium sulfite was 6.1%. Incidentally the theoretical value of the reduction ratio, calculated from the free energy change of the reaction, was 6.7%.

Comparative Example 2

The reduction of sodium sulfate was carried out in the same manner as described in Example 2 except that a catalyst formed by supporting 1% by weight of Pt on an alumina Rashig rings (cylinder), which had the same size as that of the polytetrafluoroethylene tube used in Example 2, was used. The reduction ratio was lower than 0.01%.

Example 3

An experiment for the reduction of acetone (CH$_3$COCH$_3$) in an aqueous solution was carried out.

A porous divinylbenzene-styrene copolymer was impregnated with an acetone solution of chloroplatinic acid, dried and reduced with hydrogen at 200°C to form a water-repellent catalyst containing about 0.3% by weight of platinum.

A beaker was charged with 200 ml of an aqueous solution containing 1% by weight of acetone, and 10 ml of the above catalyst was added to the solution. Hydrogen gas was circulated into the solution at a rate of 500 ml/l in the form of fine bubbles through a ball filter. Reaction was carried out at 70°C for 2 hours. The yield of isopropyl alcohol was 68%.

Example 4

Porous polytetrafluorethylene having a cylindrical shape having an outer diameter of 8 mm, an inner diameter of 5 mm and a length of 8 mm was used as a catalyst carrier. An aqueous solution of chloroplatinic acid was diluted with isopropyl alcohol and the porous polytetrafluoroethylene carrier was impregnated with the dilution. The impregnated carrier was dried and reduced in a nitrogen current at 200°C for 5 hours to obtain a water-repellent catalyst containing 0.5% by weight of platinum.

An experiment of precipitation of copper from a solution of copper nitrate was carried out in the following manner.

A vessel was charged with about 300 ml of a solution containing 1 mole/l of copper nitrate, and about 30 g of the above catalyst was thrown into the sulution. Hydrogen gas was circulated in the solution at a flow rate of 500 ml/min through a ball filter, and reaction was carried out at 50°C for 3 hours. By analysis of the residual copper concentration in the solution, it was found that more than 90% of copper contained in the solution was deposited on the water-repellent catalyst.

### Comparative Example 3

An experiment was carried out in the same manner as described in Example 4 except that porous polytetrafluoroethylene alone was used. By the naked eye observation, it was found that copper was not deposited on the polytetrafluoroethylene. Also by analysis of the copper concentration in the solution, it was confirmed that copper ions had not been reduced.

### Comparative Example 4

An experiment was carried out in the same manner as described in example 4, except that a commercially available alumina catalyst containing 0.5% by weight of platinum (hydrophilic catalyst) was used. By analysis of the copper concentration after the reaction, it was found that the ratio of reduction of the amount of copper ions was lower than 5%.

### Example 5

A porous polytetrafluoroethylene plate (2 cm × 2 cm; 1.5 mm in thickness) was impregnated with nickel by using a solution of nickel nitrate in water-propanol, and the plate was then dried and reduced at 200°C for 10 hours with hydrogen to obtain a water-repellent catalyst containing about 5% by weight of nickel.

The above catalyst was added to a solution containing 0.1 mole/l of chloroplatinic acid, and hydrogen gas was dispersed and circulated in the solution at 50°C for 2 hours. Platinum was deposited on the catalyst in an amount of 0.05 g per gram of the water-repellent catalyst. The catalyst obtained in this Example could be effectively used as an electrode of a fuel cell.

**Claims**

1. A catalystic process for reducing organic and inorganic ions and molecules which comprises contacting said ions or molecules present in an aqueous liquid reaction system in the presence of a solid hydrophobic catalyst with a reducing gaseous substrate hardly or not soluble in said aqueous liquid reaction system, characterized in that said catalyst comprises as constituents a catalyic component and, as a carrier for the catalytic component, a porous, water-repellent organic polymeric substance having fine pores and a large speific surface area and in that said catalyst has a critical surface tension of lower than $2 \cdot 10^{-2} \, N \cdot m^{-1}$ (20 dyn/cm) as measured at 20°C and a contact angle to water larger than 60°C.

2. The process according to claim 1, characterized in that the reducing gaseous substance is hydrogen, carbon monoxide or a gaseous mixture containing at least one member selected from hydrogen and carbon monoxide.

3. The process according to one of claims 1 or 2, characterized in that the catalytic component of the solid catalyst is supported on a water-repellent and oil-repellent carrier.

4. The process according to one of claims 1 to 3, characterized in that said water-repellent organic polymeric substance is selected from the group consisting of polytetrafluoroethylene, polyethylene, polystyrene, polypropylene, polymethyl methacrylate and mixtures thereof.

**Revendications**

1. Procédé catalytique visant à réduire des ions et molécules organiques et inorganiques, et qui consiste à mettre en contact lesdits ions ou molécules, présents dans un système réactionnel liquide aqueux, en présence d'une catalyseur hydrophobe solide, avec une substance gazeuse réductrice difficilement soluble ou non soluble dans ledit système réactionnel liquide aqueux, caractérisé en ce que ledit catalyseur comporte comme constituant un composant catalytique et, en tant que support du composant catalytique, une substance polymère organique poreuse hydrophobe comportant de fins pores et une surface spécifique étendue et que ledit canalyseur possède une tension de surface critique inférieure à $2.10^{-2} \, N.m^{-1}$ (20 dynes/cm) mesurée à 20°C et un angle de contact avec l'eau supérieur à 60°.

2. Procédé selon la revendication 1, caractérisé en ce que la substance gazeuse réductrice est de l'hydrogène, du monoxyde de carbone ou un mélange gazeux contenant au moins un élément choisi parmi l'hydrogène et le monoxyde de carbone.

3. Procédé selon l'une des revendications 1 ou 2, caractérisé en ce que le composant catalytique du catalyseur solide est soutenu sur un support hydrophobe et oléorésistant.

4. Procédé selon l'une des revendications 1 à 3, caractérisé en ce que ladite substance polymère organique hydrophobe est choisie dans le groupe incluant le polytétrafluoroéthylène, le polyéthylène, le polystyrène, le polypropylène, le polyméthacrylate de méthyle et des mélanges de ces substances.

**Patentanshrüche**

1. Katalytisches Verfahren zur Reduktion von organischen und anorganischen Ionen und Molekülen, bei dem diese in einem wässrigen flüssigen Reaktionssystem vorliegenden Ionen oder Moleküle

**0 0 15 585**

in gegenwart eines festen hydrophoben Katalysators mit einer reduzierenden gasförmigen Substanz in Berührung gebracht werden, die in diesem wässrigen flüsigen Reaktionssystem kaum oder nicht löslich ist, dadurch gekennzeichnet, daß dieser Katalysator als Bestandteile eine katalytische Komponente und, als Träger für die katalytische Komponente, eine poröse, wasserabweisende organische polymere Substanz, die feine Poren und eine große specifische Oberfläche aufweist, enthält und daß dieser Katalysator eine kritische Oberflächenspannung von weniger als $2 \times 10^{-2}$ N.m$^{-1}$ (20 dyn/cm), gemessen bei 20°C, und einen Kontaktwinkel gegenüber Wasser von mehr als 60° hat.

2. Verfahren nach Anspruch 1, dadurch gekennzeichnet, daß die reduzierende gasförmige Substanz Wasserstoff, Kohlenmonoxid oder ein gasförmiges Gemisch ist, das mindestens ein Mitglied aus der Gruppe Wasserstoff und Kohlenmonoxid enthält.

3. Verfahren nach einem der Ansprüche 1 oder 2, dadurch gekennzeichnet, daß die katalytische Komponente des festen Katalysators auf einem wasserabweisenden und ölabweisenden Träger aufgetragen ist.

4. Verfahren nach einem der Ansprüche 1 bis 3, dadurch gekennzeichnet, daß die wasserabweisende organische polymere Substanz aus der aus Polytetrafluorethylen, Polyethylen, Polystyrol, Polypropylen, Polymethylmethacrylat und deren Gemische bestehenden Gruppe gewält ist.